# FASCICULE DE BREVET EUROPEEN

(11) **EP 2 556 164 B1**
(45) Date de publication et mention de la délivrance du brevet: **30.05.2018**
(21) Numéro de dépôt: 11719662.6
(22) Date de dépôt: 06.04.2011
(51) Int. Cl.: C12Q 1/04, C12Q 1/06, C12M 1/34, G01B 11/24, G01N 33/487

(54) **PROCÉDÉ DE DÉTECTION D'AMAS DE PARTICULES BIOLOGIQUES.**
VERFAHREN FÜR DEN NACHWEIS VON CLUSTERN AUS BIOLOGISCHEN PARTIKELN
METHOD FOR DETECTING CLUSTERS OF BIOLOGICAL PARTICLES

(30) Priorité: 06.04.2010 FR 1001406
(43) Date de publication de la demande: 13.02.2013
(73) Titulaire: Commissariat à l'Énergie Atomique et aux Énergies Alternatives, 75015 Paris (FR); Biomérieux, 69280 Marcy L'Etoile (FR)
(72) Inventeur: MOY, Jean-Pierre, 38120 Saint Egreve (FR); DUPOY, Mathieu, 38100 Grenoble (FR); FIGADERE, Olivier, 01150 Villebois (FR); PINSTON, Frédéric, 38000 Grenoble (FR); BOSSY, Geneviève, 01500 Amberieu En Bugey (FR); DRAZEK, Laurent, 38100 Grenoble (FR); GUICHERD, Maryse, 38510 Vezeronce Curtin (FR); ROMANENS, Fabien, 38000 Grenoble (FR); MALLARD, Frédéric, 38340 Voreppe (FR)
(74) Mandataire: Gevers & Orès
(86) Numéro de dépôt international: PCT/IB2011/051481
(87) Numéro de publication internationale: WO 2011/125033

(56) Documents cités:
- WO-A1-95/00661
- WO-A1-99/24786
- WO-A1-2004/005537
- FR-A1- 2 738 343
- JP-A- 59 166 842
- US-A- 3 468 610
- US-A1- 2009 060 134
- VERRAN J ET AL: "The retention of bacteria on hygienic surfaces presenting scratches of microbial dimensions.", LETTERS IN APPLIED MICROBIOLOGY MAR 2010 LNKD- PUBMED:20070506, vol. 50, no. 3, mars 2010 (2010-03), pages 258-263, XP002601175, ISSN: 1472-765X
- WANG H ET AL: "Effect of surface roughness on retention and removal of Escherichia coli O157:H7 on surfaces of selected fruits.", JOURNAL OF FOOD SCIENCE 2009 JAN-FEB LNKD- PUBMED:19200095, vol. 74, no. 1, janvier 2009 (2009-01), pages E8-E15, XP002601176, ISSN: 1750-3841

## Description

L'invention porte sur un procédé de détection d'amas de particules biologiques, telles que des microorganismes (bactéries, levures, champignons...) ou des cellules végétales ou animales, sur une surface, par exemple la surface d'un milieu de culture ou d'un substrat fonctionnalisé. Ces particules présentent des dimensions microscopiques, typiquement de l'ordre de 0,5 µm à 3 mm, et plus particulièrement de 0,5 µm à 10 µm.

L'invention s'applique en particulier à la détection de colonies de microorganismes.

Pour de nombreuses applications, on souhaite détecter le plus tôt possible la croissance de microorganismes tels que bactéries ou levures sur un milieu de culture, le plus souvent une surface de gel nutritif. En général, la surface d'un milieu de culture gélosé s'écarte notablement d'une planéité parfaite, car elle présente fréquemment des défauts locaux de quelques micromètres (µm) de profondeur sur des distances de quelques millimètres (mm). Des poussières ou débris apportés en même temps que l'échantillon peuvent également introduire des déformations locales de haute fréquence spatiale de la surface.

Les bactéries et levures sont difficilement repérables au début de leur croissance, car leur absorption est très faible dans les domaines du visible, du proche ultraviolet et du proche infrarouge, et leur indice de réfraction très proche de celui de leur milieu environnant. Ainsi, typiquement seules les colonies ayant un diamètre supérieur à 100 µm sont détectables à l'oeil nu ; le temps nécessaire à la croissance de colonies de ces dimensions est typiquement de l'ordre de 6 à 24 heures (h).

L'examen microscopique poussé, de préférence en fond noir, est une voie possible mais difficile à mettre en oeuvre.

D'autres techniques sont couramment appliquées.

Par exemple, il est possible de rendre les microorganismes fluorescents par différents additifs non-fluorescents que le métabolisme des microorganismes transforme en une substance fluorescente, choisie pour
rester longtemps à l'intérieur des microorganismes. Cette méthode réclame un temps assez long (>5 h) avant que les bactéries ne deviennent fluorescentes et la mise au point d'un métabolite fluorogène.

De même, des milieux chromogènes permettent de visualiser les microorganismes de manière sélective, mais le même problème est rencontré: la coloration réclame un temps assez long (plusieurs heures)avant d'être visible à l'oeil nu.

De plus, toutes ces méthodes risquent d'apporter des perturbations sérieuses au métabolisme des microorganismes, alors que la suite des tests (par exemple mesure de la sensibilité aux antibiotiques) exige un développement des microorganismes, dans les conditions les plus favorables.

En outre, ces méthodes sont bien connues, et ont été largement décrites dans la littérature et en particulier dans les documents :
- Verran J. et al. : « The retention of bacteria on hygienic surfaces presenting scratches of microbial dimensions. », Lett. Appl. Microbiol. 2010 Mar; 50(3):258-63*;*
- Wang H. et al. : « Effect of surface roughness on retention and removal of Escherichia coli O157:H7 on surfaces of selected fruits. » Journal of Food Science 74(1) : E8-E15 February 2009;
- US 3 468 610 A;
- WO 95/00661 A1;
- WO 2004/005537 A1;
- US 2009/060134 A1;
- JP 59 166842 A.

L'invention vise à pallier les inconvénients précités en procurant un procédé de détection d'amas de particules biologiques aussi peu intrusif que possible et permettant la détection précoce d'amas de petites dimensions tels que des colonies de microorganismes au début de leur croissance.

Conformément à l'invention un tel but est atteint par un procédé de détection d'amas de particules biologiques sur une surface, comportant les étapes consistant à :
a. déterminer une représentation topographique de ladite surface consistant en une représentation bidimensionnelle, c'est-à-dire une image, sur laquelle la variation d'intensité entre deux pixels adjacents traduit une variation d'altitude et/ou une variation locale de l'indice de réfraction ;
b. détecter dans ladite représentation topographique au moins un contour ;
c. sélectionner parmi le ou les contours détectés au moins un contour délimitant une région susceptible de correspondre à un amas de particules biologiques.

Ces étapes sont mises en oeuvre à l'aide d'un ordinateur programmé de manière opportune ou d'un autre moyen électronique de traitement des données, associé (notamment pour l'exécution de l'étape a) à un dispositif de mesure approprié.

Selon des modes de réalisation avantageux de l'invention :
- Lesdites particules biologiques peuvent être choisies parmi des microorganismes, tels que des bactéries, levures ou champignons, et des cellules végétales ou animales.
- Lesdites particules biologiques peuvent présenter un diamètre ou une dimension principale comprise entre 10µm et 3 mm, ou, préférentiellement, inférieure ou égale à quelques centaines de 100 µm, et par exemple inférieure ou égale à 100 µm.
- Ladite surface peut être choisie parmi : l'interface entre un milieu de culture et un milieu environnant, tel que l'air, la surface d'un substrat fonctionnalisé ou la surface d'une membrane microporeuse.
- Ladite étape a. consistant à déterminer une représentation topographique de ladite surface peut être mise en oeuvre par un procédé optique qui s'effectue sans contact et sans préparation de l'échantillon afin d'assurer une intrusivité minimale. Il peut s'agir en particulier d'un procédé de microtopographie confocale chromatique ou bien de strioscopie ou d'ombroscopie.
- Ladite étape b. consistant à détecter au moins un contour peut être mise en oeuvre par mesure de la pente locale de ladite représentation topographique de la surface, avec seuillage.
- Le procédé peut comporter également une opération de prétraitement de ladite représentation topographique comportant la détection et la soustraction d'une surface de référence.
- Le procédé peut comporter la répétition des étapes a. et b. à des temps successifs, et la sélection des seules régions identifiées aux étapes b dont la forme ou la taille évolue dans le temps.

Selon une variante avantageuse de l'invention, le procédé peut comporter une étape supplémentaire c. consistant à quantifier un indicateur relatif à la taille d'un amas détecté, par exemple son volume. Cela présente notamment de l'intérêt lorsque la taille moyenne des particules biologiques d'intérêt est connue. En effet, dans ce cas, cette étape c permet d'évaluer la quantité de particules biologiques présentes dans l'amas. En effet, en connaissant la taille moyenne des particules biologiques d'intérêt, prises individuellement, il peut être aisé une fois que la taille de l'amas est déterminée, de quantifier les particules biologiques présentes dans ledit amas. Une telle variante de l'invention est particulièrement avantageuse, quand les particules biologiques sont des microorganismes et qu'un traitement subséquent sur lesdits microorganismes est envisagé, auquel cas, un prélèvement de la colonie est nécessaire. Cette variante permet alors de s'assurer que la quantité de microorganismes présents est suffisante, avant d'effectuer, par exemple, un prélèvement.

Selon une autre variante avantageuse de l'invention, le procédé peut comporter une étape supplémentaire d'identification, in situ ou ex situ, des microorganismes disposés en amas.

D'autres caractéristiques, détails et avantages de l'invention ressortiront à la lecture de la description faite en référence aux dessins annexés donnés à titre d'exemple et qui représentent, respectivement :
- La figure 1, la surface d'un milieu de culture sur laquelle sont présentes des colonies bactériennes ;
- La figure 2, une illustration schématique des différentes étapes d'un procédé selon un mode de réalisation de l'invention;
- La figure 3, une illustration du principe de la microtopographie confocale chromatique, utilisée dans une première variante du procédé selon l'invention ;
- Les figures 4A, 4B et 5A, 5B, 5C, 5D et 5E des résultats expérimentaux obtenus en appliquant la technique de microtopographie confocale chromatique à un milieu de culture ensemencé par des bactéries ;
- Les figures 6A - 6D, une deuxième variante du procédé selon l'invention, basée sur la technique de la strioscopie ; et
- Les figures 7A - 7C, une troisième variante du procédé selon l'invention, basée sur la technique de l'ombroscopie.

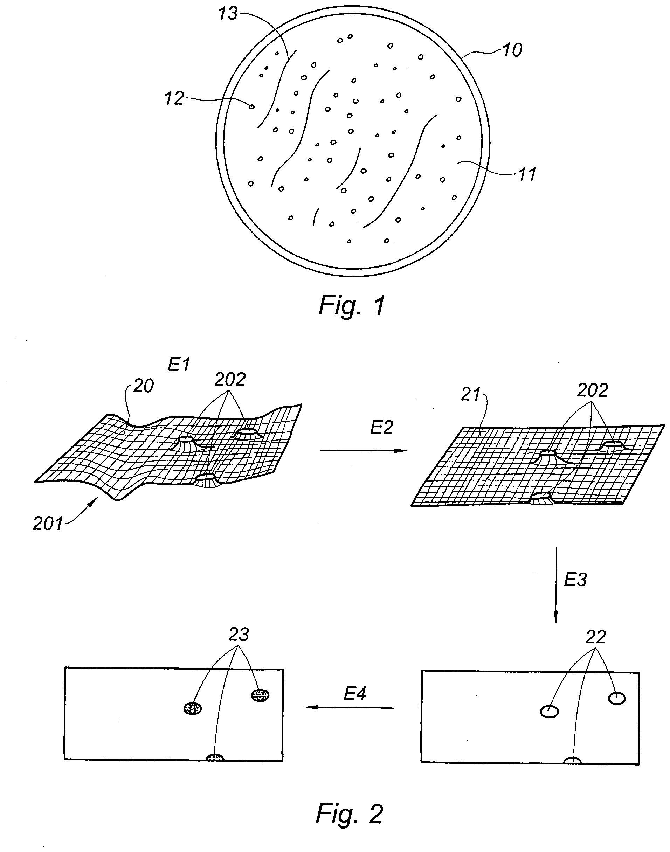

La figure 1 montre une boîte de Pétri 10 vue en plan ; cette boîte contient un milieu nutritif (une gélose), sur la surface 11 de laquelle se sont développées de colonies bactériennes 12. Le procédé de l'invention vise à détecter ces colonies à un stade précoce de leur croissance, lorsque leur diamètre est trop faible pour qu'elles soient visibles à l'oeil nu (diamètre de quelques dizaines de micromètres, par exemple 30 µm ou moins). La surface 11 du milieu de culture (c'est-à-dire l'interface gélose-air) présente des irrégularités 13, liées à l'état de surface de la gélose. Dans l'exemple de la figure 1, la gélose est directement exposée à l'air; par conséquent, elle a tendance à se dessécher, ce qui induit des modifications de sa surface, et notamment une variation de son niveau moyen. Avantageusement, un procédé de détection d'amas de particules biologiques sur une surface de gélose prend en considération ces irrégularités de la surface, ainsi que les modifications auxquelles elle est soumise.

Le procédé de l'invention exploite le fait, connu, que la multiplication des bactéries à la surface d'un milieu nutritif forme une protubérance. La hauteur de cette protubérance se mesure, dans les phases initiales de la croissance de la colonie, en centaines de nanomètres ou en micromètres. L'idée à la base de l'invention consiste à détecter les protubérances associées aux amas de particules biologiques au moyen de techniques - connues par elles-mêmes - de topographie de surface, associées à un traitement d'image adapté.

Comme le montre la figure 2, la première étape E1 du procédé de l'invention consiste à déterminer une représentation topographique 20 de la surface 11 sur laquelle se développent les amas 12 à détecter. Dans le cas représenté sur cette figure, il s'agit d'une représentation topographique tridimensionnelle, prenant en particulier la forme d'une « nappe tridimensionnelle ». Une représentation topographique tridimensionnelle associe, à chaque point de la surface, des coordonnées (x,y) dans un plan Oxy et une coordonnée z selon un axe Oz de préférence perpendiculaire au plan Oxy. Cette représentation peut prendre la forme d'une surface en trois dimensions dans un repère Ox,Oy,Oz, ou d'une matrice dont les éléments a_{i,j} correspondent à la hauteur selon z des points de coordonnées (i,j) dans un plan Oxy.

La deuxième étape E2 est une opération de prétraitement de la représentation topographique 20 comportant la détection et la soustraction d'une surface de base. En effet, comme expliqué plus haut, la surface 11 peut être mal définie et même variable dans le temps. Pour assurer une bonne détection des amas de particules, il faut déterminer une surface de base extraite par un traitement approprié des données, par exemple un filtrage spatial passe-bas., la fréquence de coupure étant typiquement de l'ordre de 0.001 µm⁻¹, et par exemple comprise entre 1/2000 µm⁻¹ et 1/500 µm⁻¹. Les rayures dues à la préparation du milieu de culture, les variations même hétérogènes de l'épaisseur et la non-planéité disparaissent.

Selon une variante plus simple, une pente moyenne est calculée au niveau d'une région de la surface, pour déterminer une surface de base plane et inclinée.

La référence 21 indique la nappe tridimensionnelle (plus généralement : la représentation topographique) filtrée, dans laquelle les irrégularités 201 de faible fréquence spatiale (dues, par exemple, aux irrégularités du milieu de culture 13) ont été éliminées par l'opération de traitement que l'on vient de décrire. Seules persistent, sur la nappe 21, les irrégularités et aspérités 202 de taille suffisamment petite et/ou contenant des fréquences spatiales suffisamment élevées. On entend par fréquence spatiales élevées des fréquences supérieures à 1/500 µm⁻¹. On pourra éliminer des composantes très hautes fréquences, c'est-à-dire les composantes correspondant à des fréquences spatiales supérieures à quelques µm⁻¹, de telles composantes correspondant à la rugosité du gel composant le milieu de culture.

La troisième étape E3 consiste à extraire des contours 22 à partir de la nappe tridimensionnelle 21. D'une manière connue en soi, les contours peuvent être détectés par des filtres de détection de contour connus de l'homme du métier, par mesure de la pente locale de la nappe suivie d'un seuillage, ou par filtrage spatial passe-bande, par exemple un filtre ayant une bande passante comprise entre quelques 1/500 µm⁻¹ et quelques 1/10 µm⁻¹. Au moins certains des contours 22 définissent des régions fermées 23 qui sont identifiées, lors de la quatrième étape E4, comme pouvant correspondre à des amas de particules biologiques. L'étape E4 peut comprendre une opération de sélection des régions fermées à prendre en considération. Par exemple, il est possible d'appliquer un critère de sélection selon lequel seulement les régions de dimensions suffisamment petites (diagonale ou diamètre de l'ordre quelques dizaines de micromètres) sont retenues comme susceptibles de représenter des amas de particules biologiques. En variante ou en combinaison, il est possible de répéter les étapes E1 - E4 à des temps successifs, et de sélectionner les seules régions 22 dont la forme ou la taille varie dans le temps. En effet, les amas de particules biologiques sont des structures « vivantes », qui évoluent, tandis que les structures non biologiques ne changent pas, ou très peu, entre deux mesures successives. Cela permet aussi de mesurer la cinétique de croissance des amas.

La première étape E1 du procédé, consistant à déterminer une représentation topographique de la surface sur laquelle se développent les amas à détecter, peut être mise en oeuvre par application de plusieurs techniques différentes de microtopographie, connues par elles-mêmes. Afin de ne pas perturber la croissance des amas à détecter, les techniques sans contact, et en particulier les techniques optiques sans préparation de l'échantillon (c'est à dire ne reposant pas sur l'utilisation de colorants, fluorophores ou leurs précurseurs) sont particulièrement préférées.

D'une manière générale, une technique de microtopographie optique comporte :
- l'éclairement par une source lumineuse de la surface dont une représentation tridimensionnelle doit être réalisée ;
- la détection, par un capteur de lumière, d'un signal, correspondant à la lumière réfléchie ou transmise par cette surface ; et
- la détermination d'une représentation topographique tridimensionnelle de la surface à partir du signal détecté.

L'éclairement de la surface peut être dirigé du milieu ambiant vers la surface. Lorsque l'éclairement n'est pas collimaté, on peut le focaliser sur la zone à mesurer. La source de lumière peut-être spatialement et/ou temporellement cohérente, ou bien être incohérente. Elle peut être polychromatique ou monochromatique. La détection peut être réalisée par un capteur d'image monodimensionnel (barrette) ou bidimensionnel (matrice), de type CMOS, CCD, photomultiplicateur, photodiode, etc. Ce capteur peut avoir une fonction spectrométrique, permettant d'analyser le spectre de la lumière détectée. Il peut aussi être couplé à un diaphragme, ou trou sténopéïque, afin de constituer un dispositif de détection confocal.

Deux techniques seront décrites en détail par la suite comme constituant des modes de réalisation préférés de l'invention. Cependant, d'autres méthodes optiques peuvent également être appliquées au procédé de l'invention, par exemple : une mise au point automatique sur la surface, par auto-collimation ou par traitement d'image ; des méthodes interférométriques et/ou holographiques ; une analyse du front d'onde en réflexion ; etc.

La première technique optique de microtopographie qui a été appliquée avec succès à la détection précoce d'amas de particules biologiques sur une surface est la microtopographie confocale chromatique. La microtopographie confocale chromatique a été mise à point par la société STIL S.A. et est décrite en détail par le document FR 2 738 343.

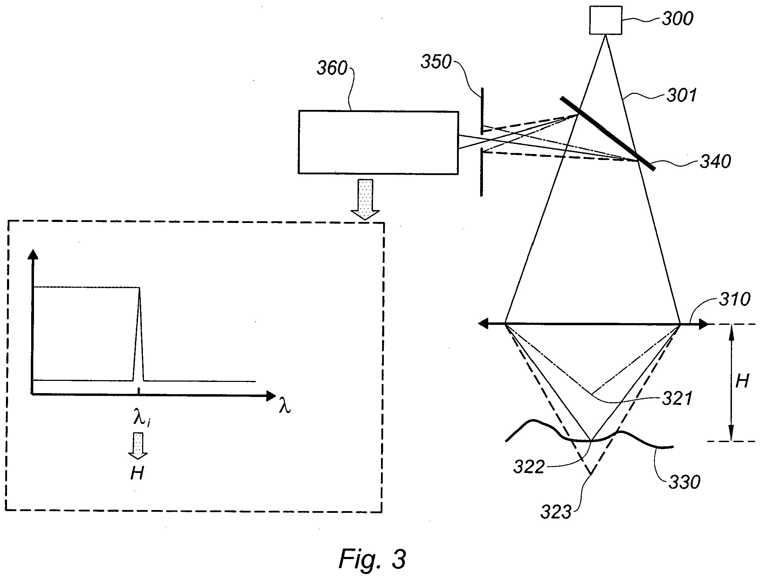

La technique de microtopographie confocale chromatique - ou imagerie confocale chromatique - est illustrée sur la figure 3

Une source ponctuelle 300 de lumière blanche (ou, en tous cas, polychromatique), par exemple réalisée à partir d'une source étendue associée à un trou sténopéïque (« pinhole » en anglais), émet un faisceau lumineux 301 qui est focalisé par un objectif 310. Cet objectif 310 présente un chromatisme axial étendu : par conséquent, les différentes composantes spectrales du faisceau 301 sont focalisées en des points focaux respectifs 321, 322, 323 ... espacés le long de l'axe optique de l'objectif. Le faisceau ainsi focalisé en incidence normale est dirigé sur la surface 330 d'un échantillon. La lumière réfléchie par la surface 330 traverse une deuxième fois l'objectif 310, cette fois en se propageant vers la source 300 ; une partie de la lumière réfléchie est extraite par un séparateur de faisceau (« beamsplitter » en anglais) 340 et dirigée vers un trou sténopéïque 350 mobile axialement, disposé devant un spectrophotomètre 360. On réalise ainsi un filtrage spatial : seuls les rayons lumineux issus d'un point conjugué du le trou sténopéïque 350 peuvent atteindre le spectrophotomètre 360. En raison de la chromaticité axiale de l'objectif 310, ces rayons présentent une longueur d'onde (λ) bien définie λᵢ, qui dépend de la distance H entre l'objectif 310 et la surface réfléchissante 330. De la mesure, réalisée par le spectrophotomètre 360, de la longueur d'onde λᵢ il est donc possible de déterminer H.

Une représentation topographique de la surface 330 peut donc être obtenue par balayage.

Une démonstration expérimentale de l'utilisation de cette technique pour la mise en oeuvre du procédé de l'invention a été réalisée, en utilisant un milieu de culture gélosé ensemencée et un système optique caractérisé par :
- une fréquence d'échantillonnage de 1 kHz ;
- une dynamique de distance d'environ 300 µm ;
- un spectrophotomètre utilisant comme détecteur une barrette CCD de 1000 pixel, chaque pixel codant une hauteur de 300 nm ; Des méthodes de traitement du signal, par exemple procédés de surpixelisation, permettent d'obtenir une résolution verticale inférieure à 300 nm. De tels procédés, connus, permettent une amélioration de la résolution en combinant le contenu de pixels adjacents.

A titre d'exemple on peut utiliser l'appareil Altisurf 500 de la société Altimet.

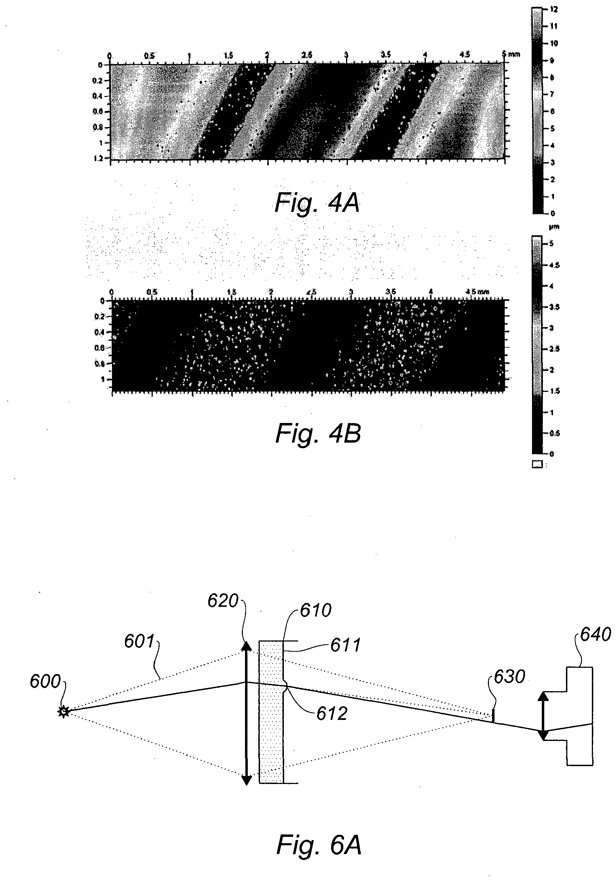

Les résultats expérimentaux sont illustrés sur les figures 4A et 4B, ainsi que 5A - 5E. Dans ces figures, la nappe tridimensionnelle a été remplacée par une représentation tout à fait équivalente exploitant une échelle de gris (gris foncé pour les faibles altitudes, gris clair ou blanc pour les altitudes plus élevées).

La figure 4A montre une représentation topographique de la surface d'un milieu de culture gélosé ensemencé avec *Staphilococcus. epidermidis.* On peut noter une distorsion de la surface de plusieurs dizaines de micromètres, sous la forme de stries. La figure 4B montre la même représentation après un prétraitement de soustraction d'une surface de base . La distorsion de surface a disparu, mais on peut distinguer sans difficulté des colonies de bactéries d'environ 2 µm de hauteur, et de diamètre compris entre 10 µm et quelques centaines de µm. Ensuite, ces colonies ont été identifiées automatiquement par calcul de la pente locale et seuillage. La taille de la surface de gélose étudiée dans cet exemple est de 1,2 x 5 mm.

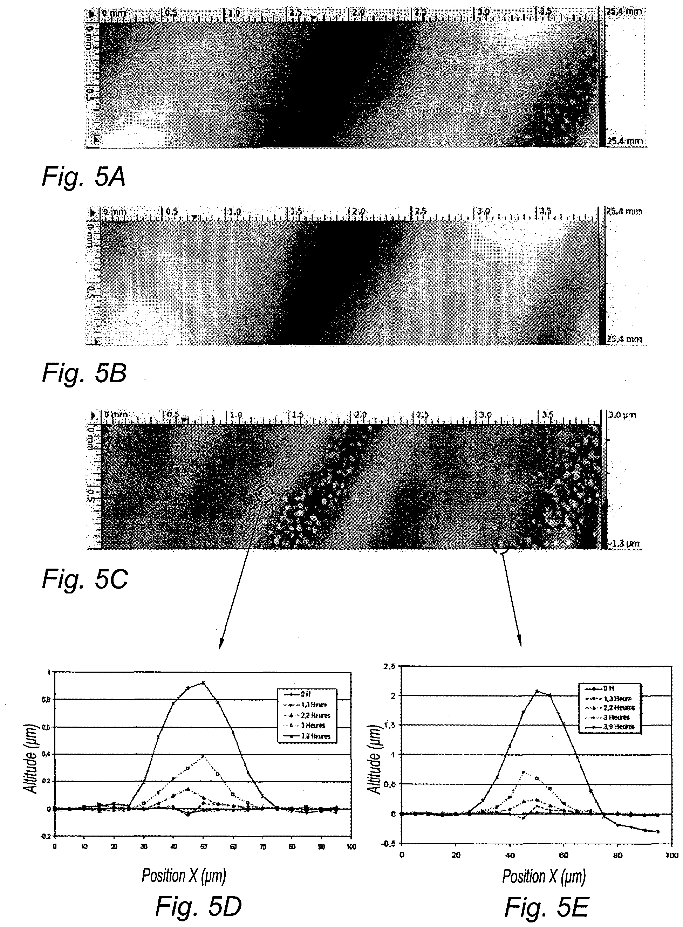

La figure 5A montre une représentation topographique de la surface d'un autre milieu de culture gélosé ensemencée avec *Escherichia Coli,* avant soustraction du fond. La figure 5B montre la surface de fond, mettant en évidence des irrégularités de surface en forme de stries, et la figure 5C la surface après soustraction du fond, sur laquelle sont clairement visibles des colonies bactériennes.

Les figures 5D et 5E montrent l'évolution dans le temps (à 0 heure ; 1,3 h ; 2,2 h ; 3 h ; 3,9 h) des profils (unidimensionnels) de deux de ces colonies. On peut voir que les colonies deviennent détectables au bout de 2h environ, et qu'il est possible de suivre leur dynamique de croissance.

Dans cette méthode, la représentation de la topographie de la surface du milieu est une représentation tridimensionnelle. Les contours détectés correspondent à des lignes fermées délimitant un volume. En fonction du procédé de détection de contour retenu, ces lignes fermées relient des pixels correspondant soit à une même pente, soit à une même altitude.

Chaque volume délimité par un contour peut-être quantifié par un indicateur, ce dernier pouvant comprendre l'intégrale du poids (c'est à dire de la hauteur) des pixels compris dans ledit volume. Lors du calcul de cette intégrale, le poids des pixels peut faire l'objet d'un seuillage. Autrement dit, on ne prend en compte que la valeur des poids dépassant un certain seuil. Ce seuil peut être établi à partir du poids d'un ou d'une pluralité de pixels constituant le contour, ou du poids d'un ou d'une pluralité de pixels répartis sur une surface délimitée par le contour.

Lorsque le type de microorganisme constituant le volume est connu, cet indicateur permet une estimation de la biomasse.

Selon un autre mode de réalisation, les amas de particules biologiques sont détectés par une méthode de strioscopie.

La strioscopie est une méthode optique, connue par elle-même, basée sur les principes de l'optique de Fourier. La figure 6A montre un montage strioscopique permettant la mise en oeuvre d'un deuxième mode de réalisation de l'invention.

Une source ponctuelle 600 émet un faisceau divergent 601 de lumière sensiblement monochromatique qui est ensuite focalisé par un objectif 620. Une boîte de Pétri transparente 610 contenant un milieu de culture ensemencé sur la surface 611 duquel se développent des colonies 612 de microorganismes à détecter est disposée immédiatement en aval de l'objectif 620, de manière à être traversée par le faisceau focalisé. Un cache 630 est disposé dans le plan focal de l'objectif 620. Ce cache occulte tous les rayons lumineux qui proviennent de la source, à travers l'objectif et la boîte de Pétri, sans avoir été déviés. Si la surface 611 était parfaitement plane et homogène (et en approximation d'optique géométrique), toute la lumière du faisceau 601 serait interceptée par le cache. En réalité, les irrégularités de la surface 611 - y comprises celles dues aux colonies 612, dévient certains rayons lumineux, qui passent ainsi à côté du cache. Du point de vue de l'optique de Fourier on sait que dans le plan focal de l'objectif se forme une distribution lumineuse correspondant au spectre de fréquences spatiales de la surface 611 ; le cache réalise ainsi un filtrage spatial passe-haut.

En aval du cache est positionné un système imageur (appareil photographique 640) mis au point sur la surface du milieu de culture. L'image acquise par le système imageur contient une information sur la pente locale de la surface 611, et permet donc de reconstituer une nappe tridimensionnelle de ladite surface (qui, de plus, est déjà partiellement filtrée passe-haut, sans besoin de prétraitement).

Etant donné que le cache a une dimension finie, seuls les rayons lumineux qui sont déviés d'un angle supérieur ou égal à une valeur seuil sont détectés. Cette valeur seuil est minimisée lorsque le cache a la taille de la tache de diffraction de la lentille. Si on suppose que la source 600 est réellement ponctuelle (ce qui est toujours une approximation), seules pourront être détectées les pentes locales supérieures à λ/(n-1)d où λ est la longueur d'onde de la lumière utilisée, d est le diamètre de la lentille et n l'indice optique du milieu. En effet, une pente locale d'angle α dévie les rayons de l'angle (n-1) α et seules les déviations supérieures à la diffraction peuvent être observées. En pratique, les milieux de culture ont des défauts de surface de quelques milliradians, ce qui impose un cache bien plus grand que cette limite théorique si on veut pouvoir se passer de tout prétraitement. Le diamètre du cache devra être de l'ordre de (n-1) I * pₘₐₓ, pₘₐₓ étant la pente locale maximale des défauts de surface, et I est la distance entre la lentille et le cache. Une micro-colonie d'épaisseur 1µm et de diamètre 30 µm impose à la surface d'onde une pente locale moyenne de (1,35-1)/15= 23 milliradians, très supérieure aux défauts de surface du gel, et est donc facilement visible. La pente locale au niveau du bord de la colonie est encore plus élevée, typiquement d'au moins quelques centaines de milliradians (ou dixièmes de radians).

Un avantage de la strioscopie sur les techniques de cartographie géométrique, telles que l'imagerie confocale chromatique, est qu'il n'est pas nécessaire d'avoir une résolution latérale meilleure que la dimension des micro-colonies : puisqu'on est en fond noir, on détecte et on localise sans aucune difficulté des objets plus petits que la limite de résolution (mais on ne sépare évidemment pas deux objets plus proches que ladite limite de résolution).

Un autre avantage est qu'avec une seule acquisition, le champ observé peut couvrir une région plus étendue qu'avec la technique de microtopographie confocale précédemment décrite. Ainsi, une acquisition d'une image par un procédé de strioscopie permet d'observer partie étendue d'une boite de Pétri, voire la totalité de cette boite.

Selon cette méthode, la représentation de la topographie de la surface du milieu est une représentation bidimensionnelle, c'est-à-dire une image, sur laquelle la variation d'intensité entre deux pixels adjacents traduit une variation d'altitude de la surface objet éclairée et/ou une variation locale de l'indice de réfraction. Les contours détectés correspondent à des lignes fermées délimitant une surface. Ces lignes relient des pixels correspondant à une même variation du niveau de gris.

Lorsque la variation d'altitude, entre deux pixels adjacents est produite par une colonie bactérienne, son bord forme avec la surface du milieu de culture un l'angle typiquement supérieur à quelques dixièmes de radians. Une telle pente est supérieure à celle formée par les variations de niveaux du milieu de culture, dont l'angle est typiquement inférieur à 10 milliradians.

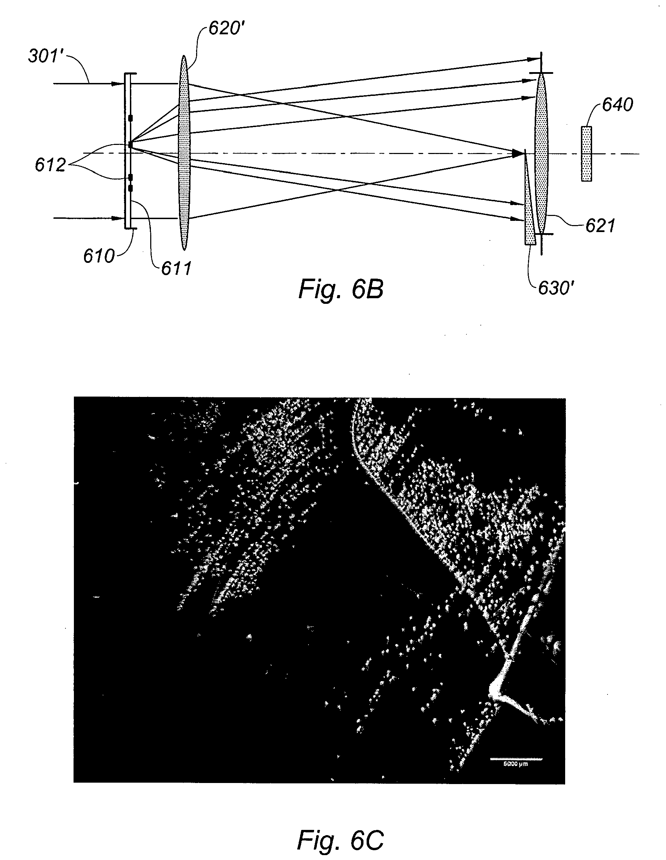

La figure 6B illustre un autre montage permettant la mise en oeuvre du procédé de l'invention par strioscopie. Contrairement au cas de la figure 6A, le faisceau d'éclairage 301' est collimaté lorsqu'il traverse la boîte de Pétri transparente 610 puis est focalisé par une première lentille 620' en aval de cette dernière (longueur focale de la lentille : 200 mm ; diamètre : 50 mm - ces valeurs sont données à titre d'exemple uniquement). Une deuxième lentille 621 (longueur focale : 10 mm : ouverture numérique ON : f/3 - là encore, ces valeurs sont données à titre d'exemple uniquement) forme une image de la boîte de Pétri. Le cache 630', disposé dans le plan focal de la lentille 620', intercepte les rayons lumineux qui n'ont pas été déviés par des variations de pente locale au niveau de la surface observée 611, ou par des variations locales de l'indice de réfraction au niveau de cette surface (ainsi que ceux qui ont été déviés vers le bas, bien que cela ne soit pas essentiel). Lorsqu'une colonie bactérienne se développe à la surface 611 du milieu de culture, elle dévie le rayonnement lumineux qui lui est incident, de sorte que ce dernier ne converge pas vers le cache 630', mais est détecté par le détecteur 640 (par exemple, une caméra de 1400x1000 pixels de 3µm chacun). Ainsi, les colonies bactériennes 612, parce qu'elles génèrent localement une fluctuation de pente au niveau de la surface 611, peuvent être détectées par le détecteur 640, sous la forme de points lumineux sur fond noir.

L'utilisation d'un faisceau d'éclairage parallèle, au lieu du faisceau divergent/convergent du montage de la figure 6A, a pour effet de rendre l'intensité du signal indépendant de la position de la portion de surface observée. En d'autres termes, sur tout le champ observé, une même pente locale au niveau de la surface observée produit un signal de même intensité sur le détecteur, indépendamment de la position de la pente locale sur la surface 611 du milieu de culture 610.

La figure 6C correspond à l'observation, avec le montage de la figure 6B, de colonies bactériennes de type Escherichia Coli, 8 heures après leur ensemencement à la surface d'une gélose TSA (Trypcase Soja Gelose). Les points blancs correspondent à des colonies bactériennes. La ligne continue formant un angle droit lumineux, dans la partie inférieure droite de l'image, correspond à une lame de verre, formant un repère visuel.

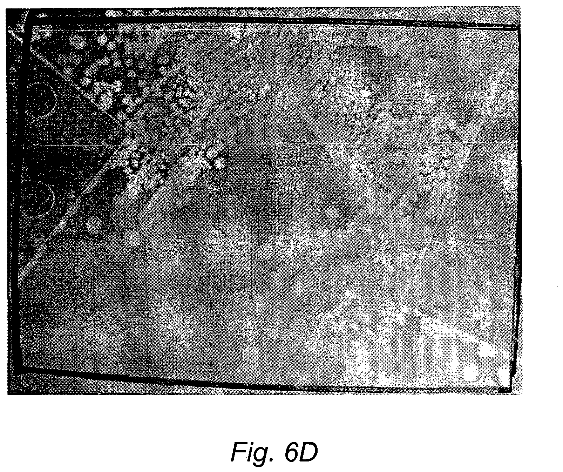

La figure 6D présente une photographie du même milieu de culture, 24 heures après l'ensemencement. On peut apprécier la bonne cohérence entre les colonies bactériennes photographiées sur la figure 6D et celles détectées, par strioscopie de transmission, sur la figure 6C.

La strioscopie peut également être mise en oeuvre selon une géométrie en réflexion, la source de lumière étant disposée face au milieu de culture, de même que le détecteur. Cela convient à l'observation de milieux opaques.

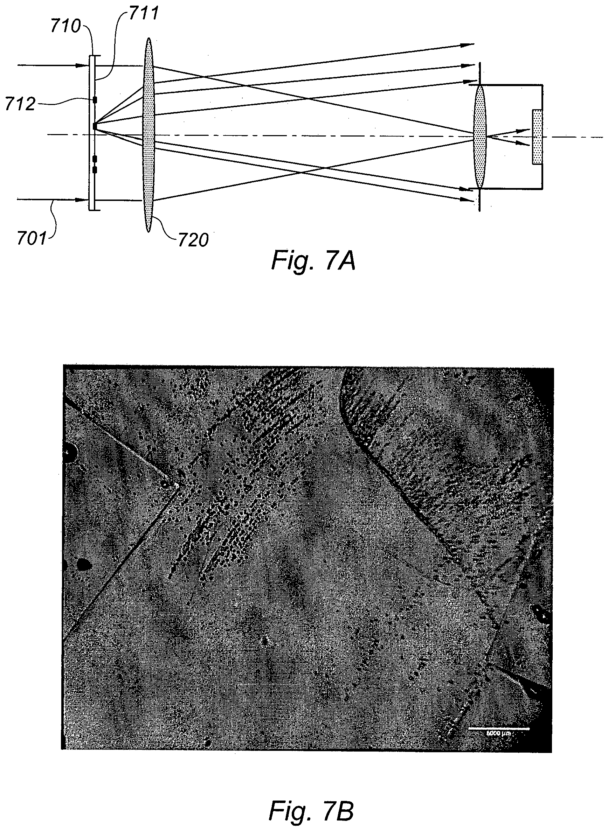

Selon un autre mode de réalisation, les colonies bactériennes sont observées par un montage mettant en oeuvre la technique d'ombroscopie. Un tel montage est représenté sur la figure 7A. Un faisceau lumineux parallèle traverse la boîte de Pétri 710 contenant le milieu de culture, puis est focalisé vers un détecteur 740 par une première lentille 720 (longueur focale de la lentille : 200 mm ; diamètre : 50 mm - ces valeurs sont données à titre d'exemple uniquement). Lorsqu'une colonie bactérienne 712 se développe à la surface 711 du milieu de culture, elle dévie le rayonnement lumineux, du fait de la variation d'altitude, et donc de la pente locale, formée à la surface 711. Contrairement à la méthode de strioscopie, le rayonnement dévié par une variation d'altitude, et/ou une variation d'indice, sur la surface observée, n'est pas capté par le détecteur 740 (caméra de 1400x1000 pixels de 3µm chacun, objectif de longueur focale f=10 mm, ouverture numérique ON=f/3 - ces valeurs sont données à titre d'exemple uniquement). Ce dispositif permet de détecter une pente locale sur une surface plane à analyser, se traduisant par l'apparition d'une zone sombre sur l'image formée par le détecteur 740. Ainsi, selon cette méthode, les colonies bactériennes 712 apparaissent sous la forme de taches sombres sur un fond clair, ce dernier correspondant à la surface 711 du milieu de culture.

Comme précédemment évoqué au sujet de la strioscopie, lors de la mise en oeuvre de l'ombroscopie, la représentation de la topographie de la surface du milieu est une représentation bidimensionnelle, c'est-à-dire une image, sur laquelle la variation d'intensité entre deux pixels adjacents traduit une variation d'altitude de la surface objet éclairée. Elle peut également traduire une variation locale d'indice au niveau de cette surface.

La figure 7B présente l'observation, avec le montage de la figure 7A, de colonies bactériennes de type Escherichia Coli 8 heures après leur ensemencement à la surface de gélose TSA. Les points sombres correspondent aux colonies bactériennes. On remarquera la cohérence de la localisation de ces colonies avec les résultats de la figure 6D (photographie) et de l'observation, en strioscopie de transmission, représentée sur la figure 6C.

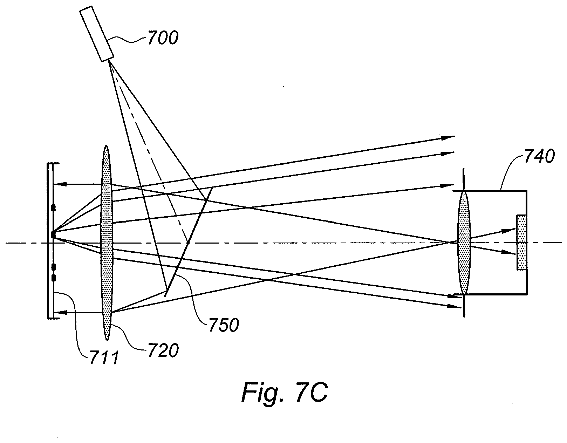

De même que la technique de strioscopie, l'observation de colonies bactériennes par ombroscopie peut être mise en oeuvre selon une géométrie de réflexion, la source de lumière 700 étant disposée en face de la boîte de Pétri 710 contenant le milieu de culture à observer, de même que le détecteur 740. La figure 7C représente un tel montage, le rayonnement produit par la source de lumière étant dévié par une lame semi-réfléchissante 750, puis rendu parallèle par la lentille 720. Un tel montage sera préféré lorsque le milieu de culture est opaque.

Aussi bien dans le cas de la strioscopie qu'en cartographie de surface, on peut avantageusement mettre en oeuvre toutes les méthodes de traitement d'image et de soustraction d'images consécutives capables de mettre en évidence des modifications dues à la croissance.

Une fois la localisation de l'amas réalisé, une identification des bactéries peut-être réalisée par une mesure connue, par exemple par analyse in-situ (diffraction, spectrométrie Raman) ou par d'autres moyens d'analyse, tels que la spectrométrie de masse. Dans ce dernier cas, l'analyse est faite *ex-situ* et nécessite donc de prélever la colonie de bactéries.

La localisation par topographie peut par exemple permettre de sélectionner des amas dont le volume dépasse un certain seuil, les amas sélectionnés faisant ensuite l'objet d'une analyse qualitative

L'invention n'est pas limitée à la détection de colonies de microorganismes sur la surface d'un milieu de culture. La surface sur laquelle se trouvent les amas biologiques, et plus généralement les particules biologiques, à détecter peut aussi être un solide, par exemple, un substrat en verre ou silicium fonctionnalisé, ou un filtre microporeux utilisé pour récupérer des bactéries contenues dans un liquide filtré et pouvant par exemple, mais non nécessairement, être placé sur un milieu de culture. Les opérations de prétraitement mises en oeuvre lors de la deuxième étape E2 du procédé seront de préférence adaptées au type de surface considéré.

Le milieu ambiant, situé au-dessus de la surface, peut être du vide ou un fluide tel qu'un gaz ou un liquide, le gaz et en particulier l'air étant le mode préféré. Le milieu ambiant peut être confiné, ce qui évite les risques de contamination et, le cas échéant, d'évaporation de la gélose.

## Revendications

1. Procédé de détection d'amas de particules biologiques (12) formant des protubérances sur une surface (11), **caractérisé en ce qu'**il comporte les étapes consistant à :
a. déterminer (E1) une représentation topographique (20) de ladite surface consistant en une représentation bidimensionnelle, c'est-à-dire une image, sur laquelle la variation d'intensité entre deux pixels adjacents traduit une variation d'altitude et/ou une variation locale de l'indice de réfraction ;
b. détecter (E3) dans ladite représentation topographique au moins un contour ;
c. sélectionner (E4) parmi le ou les contours détectés au moins un contour délimitant une région susceptible de correspondre à un amas de particules biologiques ;
ces étapes étant mises en oeuvre à l'aide d'un moyen électronique de traitement des données, lesdites particules biologiques étant choisies parmi des microorganismes.

2. Procédé selon la revendication 1 dans lequel lesdits microorganismes sont choisis parmi des bactéries, des levures ou des champignons.

3. Procédé selon l'une des revendications précédentes dans lequel lesdites particules biologiques présentent un diamètre ou une dimension principale inférieure ou égale à 100 µm.

4. Procédé selon l'une des revendications précédentes dans lequel ladite surface (11) est choisie parmi : l'interface entre un milieu de culture et un milieu environnant, tel que l'air ; la surface d'un substrat fonctionnalisé ou la surface d'une membrane microporeuse.

5. Procédé selon la revendication 4, dans lequel lesdits amas de particules biologiques sont des colonies bactériennes en croissance sur un milieu de culture gélosé.

6. Procédé selon l'une des revendications précédentes dans lequel ladite étape a. consistant à déterminer une représentation topographique de ladite surface est mise en oeuvre par un procédé optique effectué sans contact et sans préparation de l'échantillon.

7. Procédé selon la revendication 6 dans lequel ladite étape a. consistant à déterminer une représentation topographique de ladite surface est mise en oeuvre par microtopographie confocale chromatique.

8. Procédé selon l'une quelconque des revendications 5 à 7, comportant en outre une étape de prétraitement de la représentation topographique consistant à conserver les fréquences spatiales comprises entre 1/2000 µm⁻¹ et 1/500 µm⁻¹.

9. Procédé selon l'une quelconque des revendications 5 à 8, comportant en outre, préalablement à l'étape b., une étape de prétraitement de la représentation topographique consistant à :
- calculer une image numérique de référence par application d'un filtre spatial passe-bas à l'image numérique, ledit filtre spatial ayant une fréquence de coupure de l'ordre de 0.001 µm⁻¹, par exemple comprise entre 1/2000 µm⁻¹ et 1/500 µm⁻¹ ;
- soustraire l'image numérique de référence de la représentation topographique.

10. Procédé selon l'une quelconque des revendications 5 à 9, comportant, préalablement à l'étape b., une étape de prétraitement de la représentation topographique consistant à filtrer les fréquences spatiales supérieures à quelques µm⁻¹.

11. Procédé selon la revendication 6 dans lequel ladite étape a. consistant à déterminer une représentation topographique de ladite surface est mise en oeuvre par strioscopie ou par ombroscopie.

12. Procédé selon l'une des revendications précédentes dans lequel ladite étape b. consistant à détecter au moins un contour est mise en oeuvre par mesure de la pente locale de ladite représentation topographique de la surface, avec seuillage.

13. Procédé selon l'une des revendications précédentes comportant également une opération de prétraitement (E2) de ladite représentation topographique comportant la détection et la soustraction d'une surface de référence.

14. Procédé selon l'une des revendications précédentes comportant la répétition des étapes a. et b. à des temps successifs, et la sélection des seules régions identifiées aux étapes b. dont la forme ou la taille évolue dans le temps.

15. Procédé selon l'une des revendications précédentes, comportant une étape supplémentaire d. consistant à évaluer la quantité de particules biologiques présentes dans l'amas.

16. Procédé selon l'une des revendications 2 à 14, qui comprend une étape supplémentaire d'identification, in situ ou ex situ, des particules disposées en amas.

## Patentansprüche

1. Verfahren zum Nachweis von Clustern von biologischen Partikeln (12), die Vorsprünge auf einer Oberfläche (11) bilden, **dadurch gekennzeichnet, dass** es die Schritte umfasst, die aus Folgendem bestehen:
a. Bestimmen (E1) einer topographischen Darstellung (20) der Oberfläche, bestehend aus einer zweidimensionalen Darstellung, d. h. einem Bild, auf dem die Variation der Intensität zwischen zwei benachbarten Pixeln eine Höhenvariation und/oder eine lokale Variation des Brechungsindexes übersetzt;
b. Nachweisen (E3) in der topographischen Darstellung mindestens einer Kontur;
c. Auswählen (E4) aus der oder den nachgewiesenen Kontur (en), mindestens einer Kontur, die eine Region begrenzt, die einem Cluster von biologischen Partikeln entsprechen kann;
wobei diese Schritte mit Hilfe eines elektronischen Mittels zur Verarbeitung von Daten durchgeführt wird, wobei die biologischen Partikel aus Mikroorganismen ausgewählt sind.

2. Verfahren nach Anspruch 1, wobei die Mikroorganismen ausgewählt sind aus Bakterien, Hefen oder Pilzen.

3. Verfahren nach einem der vorhergehenden Ansprüche, wobei die biologischen Partikel einen Durchmesser oder eine Hauptabsmessung von weniger als oder gleich wie 100 µm aufweisen.

4. Verfahren nach einem der vorhergehenden Ansprüche, wobei die Oberfläche (11) ausgewählt ist aus: der Schnittstelle zwischen einem Kulturmedium und einem umgebenden Medium wie z. B. Luft; der Oberfläche eines funktionalisierten Substrats oder der Oberfläche einer mikroporösen Membran.

5. Verfahren nach Anspruch 4, wobei die Cluster von biologischen Partikeln Bakterienkolonien in Wachstum auf einem Agar-Kulturmedium sind.

6. Verfahren nach einem der vorhergehenden Ansprüche, wobei der Schritt a., der darin besteht, eine topographische Darstellung der Oberfläche zu bestimmen, durch einen optischen Prozess durchgeführt wird, der ohne Kontakt und ohne Zubereitung der Probe ausgeführt wird.

7. Verfahren nach Anspruch 6, wobei der Schritt a., der darin besteht, eine topographische Darstellung der Oberfläche zu bestimmen, durch chromatische Konfokalmikrotopographie durchgeführt wird.

8. Verfahren nach einem der Ansprüche 5 bis 7, umfassend außerdem einen Schritt des Vorbehandelns der topographischen Darstellung, der darin besteht, die räumlichen Frequenzen, die zwischen 1/2000 µm⁻¹ und 1/500 µm⁻¹ liegen, zu aufrechtzuerhalten.

9. Verfahren nach einem der Ansprüche 5 bis 8, umfassend außerdem vor dem Schritt b., einen Schritt des Vorbehandelns der topographischen Darstellung, die darin besteht:
- ein numerisches Referenzbild durch Anwenden eines räumlichen Tiefpassfilters auf das numerische Bild zu berechnen, wobei der räumliche Tiefpassfilter eine Grenzfrequenz im Bereich von 0,001 µm⁻¹, z. B. zwischen 1/2000 µm⁻¹ und 1/500 µm⁻¹, aufweist;
- Subtrahieren des numerischen Referenzbildes von der topographischen Darstellung.

10. Verfahren nach einem der Ansprüche 5 bis 9, umfassend vor dem Schritt b., einen Schritt des Vorbehandelns der topographischen Darstellung, die darin besteht, die räumlichen Frequenzen von mehr als einigen µm⁻¹ zu filtern.

11. Verfahren nach Anspruch 6, wobei der Schritt a., der darin besteht, eine topographische Darstellung der Oberfläche zu bestimmen, durch Strioskopie oder durch Ombroskopie durchgeführt wird.

12. Verfahren nach einem der vorhergehenden Ansprüche, wobei der Schritt b., der darin besteht, mindestens eine Kontur nachzuweisen, durch Messung der lokalen Neigung der topographischen Darstellung der Oberfläche mit Schwellenwertverfahren durchgeführt wird.

13. Verfahren nach einem der vorhergehenden Ansprüche, umfassend ebenfalls einen Vorgang zur Vorbehandlung (E2) der topographischen Darstellung, umfassend den Nachweis und die Subtraktion einer Referenzoberfläche.

14. Verfahren nach einem der vorhergehenden Ansprüche, umfassend die Wiederholung der Schritte a. und b. zu aufeinander folgenden Zeiten, und die Auswahl nur der Regionen, die in den Schritten b. identifiziert wurden, deren Form oder Größe sich im Laufe der Zeit entwickelt.

15. Verfahren nach einem der vorhergehenden Ansprüche, umfassend einen zusätzlichen Schritt d., der darin besteht, die Menge der biologischen Partikel zu bewerten, die im Cluster vorhanden ist.

16. Verfahren nach einem der Ansprüche 2 bis 14, das einen zusätzlichen Schritt des Identifizierens in *situ* oder *ex situ* der Partikel umfasst, die im Cluster angeordnet sind.

## Claims

1. Method for detecting clusters of biological particles (12) forming protuberances on a surface (11), **characterised in that** it comprises the steps consisting of:
a. determining (E1) a topographical representation (20) on said surface consisting of a two-dimensional representation, that is to say an image, on which the variation in intensity between two adjacent pixels represents a variation in altitude and/or a local variation in the refractive index;
b. detecting (E3) at least one contour in said topographical representation;
c. selecting (E4), from the contour or contours detected, at least one contour detecting a region liable to correspond to a cluster of biological particles;
these steps being implemented by means of an electronic data processing means, said biological particles being chosen from microorganisms.

2. Method according to claim 1, in which said microorganisms are chosen from bacteria, yeasts or fungi.

3. Method according to any of the preceding claims, in which said biological particles have a diameter or a principal dimension less than or equal to 100 µm.

4. Method according to any of the preceding claims, in which said surface (11) is chosen from: the interface between a culture medium and a surrounding medium, such as the air; the surface of a functionalised substrate or the surface of a microporous membrane.

5. Method according to claim 4, in which said clusters of biological particles are bacterial colonies growing on a gelose culture medium.

6. Method according to any of the preceding claims, in which said step a. consisting of determining a topographical representation of said surface is implemented by means of an optical method carried out without contact and without preparation of the sample.

7. Method according to claim 6, in which said step a. consisting of determining a topographical representation of said surface is implemented by chromatic confocal microtopography.

8. Method according to any of claims 5 to 7, further comprising a step of pretreatment of the topographical representation consisting of preserving the spatial frequencies lying between 1/2000 µm⁻¹ and 1/500 µm⁻¹.

9. Method according to any of claims 5 to 8, further comprising, prior to step b., a step of pretreatment of the topographical representation consisting of:
- calculating a reference digital image by applying a low-pass spatial filter to the digital image, said spatial filter having a cutoff frequency of around 0.001 µm⁻¹, for example between 1/2000 µm⁻¹ and 1/500 µm⁻¹ ;
- subtracting the reference digital image from the topographical representation.

10. Method according to any of claims 5 to 9, comprising, prior to step b., a step of pretreatment of the topographical representation consisting of filtering the spatial frequencies greater than a few µm⁻¹.

11. Method according to claim 6, in which said step a. consisting of determining a topographical representation of said surface is implemented by strioscopy or by ombroscopy.

12. Method according to any of the preceding claims, in which said step b. consisting of detecting at least one contour is implemented by measuring the local slope of said topographical representation of the surface, with thresholding.

13. Method according to any of the preceding claims, also comprising an operation of pretreatment (E2) of said topographical representation comprising the detection and subtraction of a reference surface.

14. Method according to any of the preceding claims, comprising the repetition of steps a. and b. at successive times, and the selection solely of the regions identified at steps b. where the shape and size change over time.

15. Method according to any of the preceding claims, comprising a supplementary step d. consisting of evaluating the quantity of biological particles present in the cluster.

16. Method according to any of claims 2 to 14, which comprises a supplementary step of identification, *in situ* or ex *situ,* of the particles disposed in a cluster.
